# EUROPÄISCHE PATENTSCHRIFT

(11) **EP 0 454 002 B1**
(45) Veröffentlichungstag und Bekanntmachung des Hinweises auf die Patenterteilung: **28.06.1995**
(21) Anmeldenummer: 91106384.0
(22) Anmeldetag: 20.04.1991
(51) Int. Cl.: C07C 403/12

(54) **Dehydratisierungsverfahren**
Dehydration process
Procédé de déshydratation

(30) Priorität: 27.04.1990 CH 1443/90
(43) Veröffentlichungstag der Anmeldung: 30.10.1991
(73) Patentinhaber: F. HOFFMANN-LA ROCHE AG, 4002 Basel (CH)
(72) Erfinder: Mazzuchelli, Mario, CH-4056 Basel (CH); Soukup, Milan, Dr., CH-4303 Kaiseraugst (CH); Spurr, Paul, Dr., CH-4057 Basel (CH); Stritt, Claude, CH-4056 Basel (CH)
(74) Vertreter: Urech, Peter, Dr.

(56) Entgegenhaltungen:
- CH-A- 262 181
- GB-A- 1 173 063
- CHEMICAL ABSTRACTS, vol. 102, 1985, Columbus, Ohio, US;abstract no. 132301, LOMJANSKY, J. ET AL.: 'Vitamin A acetate.' Seite 654; Spalte 2 ;
- CHEMICAL ABSTRACTS, vol. 74, 1971, Columbus, Ohio, US; abstract no. 111210, TSUKURIMICHI; E.: 'Measurement of catalytic activities of anhydrous metal bromides for the intramolecular dehydration of tert-butyl alcohol or tert-pentyl alcohol.' Seite 341; Spalte 1;
- abstract no. 111210, TSUKURIMICHI, E.: 'Measurement of catalytic activities ofanhydrous metal bromides for the intramolecular dehydration of tert-butylalcohol or tert-pentyl alcohol.' Seite 341 ;Spalte 1 ;

## Beschreibung

Die vorliegende Erfindung betrifft ein neues Verfahren zur Herstellung von Cycloalkenylalkenen mit durchkonjugierter Struktur, die insbesondere als Zwischenprodukte in Carotinoidsynthesen geeignet sind.

Das erfindungsgemässe Verfahren ist dadurch gekennzeichnet, dass man 1-(5-Acetoxy-3-methyl-penta-1,3-dienyl) -2,6,6-trimethyl-cyclohexanol Ia oder 4-Acetoxy-1-(5-acetoxy-3-methyl-penta-1,3-dienyl) -2,6,6-trimethyl-cyclohexanol Ib oder 1-(9-Acetoxy-3,7-dimethyl-4-hydroxy-nona-2,5,7-trienyl) -2,6,6-trimethyl-cyclohexen Ic in Gegenwart eines Erdalkalimetallbromids oder von Manganbromid, oder eines Hydrates eines solchen Bromids, als Katalysator sowie in einem organischen Lösungsmittel dehydratisiert.

Dieses Verfahren wird anhand folgender Reaktionsschemata veranschaulicht: 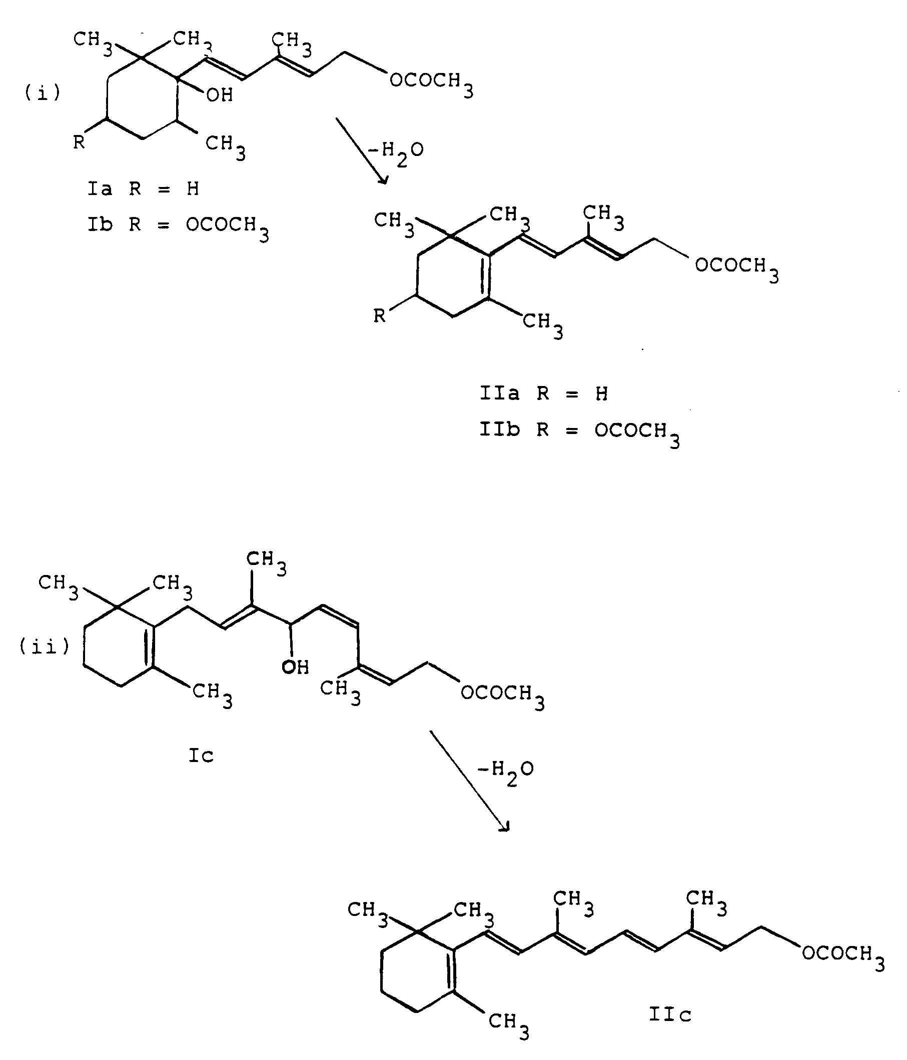
GB-A 1 173 063 beschreibt die Dehydratisierung von 5-(1-Hydroxy-4-acyloxy/oxo-2,2,6-trimethyl-cyclohexyl)-1-acyloxy-3-methyl-pent-2-en-4-in (X) zum entsprechenden Cyclohex-6-enderivat (XI), und zwar unter Verwendung von p-Toluolsulfonsäure oder Jod als Dehydratisierungsmittel und von Benzol oder Toluol als Lösungsmittel. Diese Dehydralisierung erfolge auch unter Verwendung eines Phosphoroxyhalogenids, z.B. POCl₃, in Gegenwart eines Säurebindemittels, z.B. Chinolin. Auch in dieser Patentpublikation beschrieben wird die simultane Acylierung und Dehydratisierung des 5-(1,4-Dihydroxy-2,2,6-trimethyl-cyclohexyl)-3-methylpent-2-en-4-ins sowie des entsprechenden 4-Acyloxy- oder 4-Oxocyclohexanderivats zum Cyclohex-6-enderivat XI (siehe oben), und zwar unter Verwendung eines Gemisches einer C₁₋₇-Alkansäure mit dem entsprechenden Säureanhydrid. Diese Angaben sind der Seite 3, Zeilen 5-10 und 18 - 23, der Seite 4, Zeilen 10 - 15 und 32-36, und der Seite 5, Zeilen 22-28, der GB-Patentpublikation zu entnehmen.

In Chem.Abs. 102, Seite 654, 132 301 h (1985) [Tschechoslowakische Patentschrift 205.884] wird die simultane Dehydratisierung und Umlagerung der Vitamin-A-Acetat-Vorstufe 1-(9-Acetoxy-3,7-dimethyl-4-hydroxy-nona-2,5,7-trienyl)-2,6,6-trimethyl-cyclohexen beschrieben, wobei Salzsäure als Dehydratisierungsmittel, Methylenchlorid als Lösungsmittel und Temperaturen bis 0°C verwendet werden. Weitere Reaktionsbedingungen für diese simultane Dehydratisierung und Umlagerung sind in Helv. Chim. Acta XXXII, Fasc. II, Nr. 63, 489 - 499 (1949), in Pure & Applied Chem. 51, 447 - 462 (1979) sowie in der schweizerischen Patentschrift Nr. 262 181 beschrieben.

Gegenüber dem obenerwähnten Stand der Technik hat es sich gezeigt, dass das erfindungsgemässe Verfahren ökonomisch ist, zumal dabei aufwendig zu entfernende und zu beseitigende Nebenprodukte und Abfallprodukte vermieden werden können und das Verfahren gegenüber bisher verwendeten Dehydratisierungsverfahren mit nur 5-10 Molprozent Katalysator mit guter Ausbeute abläuft.

Unter Erdalkalimetallbromiden sind Magnesiumbromid, Calciumbromid, Strontiumbromid und Bariumbromid zu verstehen, von denen Magnesiumbromid, Calciumbromid und Strontiumbromid, insbesondere die ersten beiden, bevorzugt sind.

Geeignete organische Lösungsmittel zur Durchführung des erfindungsgemässen Verfahrens sind Nitrile, z.B. Acetonitril; Ketone, z.B. Aceton; sowie Ester, z.B. Essigsäure-methylester oder -isopropenylester. Die bevorzugten Lösungsmittel sind Acetonitril, Aceton und Essigsäure-isopropenylester. Als Katalysatoren in Hydratform eignen sich beispielsweise Calciumbromid-Dihydrat (CaBr₂·2H₂O), Magnesiumbromid-Hexahydrat (MgBr₂·6H₂O), Manganbromid-Tetrahydrat (MnBr₂·4H₂O) und Strontiumbromid-Hexahydrat (SrBr₂·6H₂O). Bevorzugte Katalysatoren sind Calciumbromid, Magnesiumbromid und Manganbromid sowie Hydrate davon (insbesondere CaBr₂·2H₂O und MgBr₂·6H₂O).

Zum Erreichen optimaler Reaktionsbedingungen hängt die Wahl des Katalysators im allgemeinen von dem zu verwendenden Lösungsmittel ab, d.h. gewisse Katalysator/Lösungsmittel-Kombinationen sind im erfindungsgemässen Verfahren besonders vorteilhaft. Besonders bevorzugte Katalysator/Lösungsmittel-Kombinationen sind CaBr₂·2H₂O/Acetonitril, CaBr₂·2H₂O/Aceton und MgBr₂·6H₂O/Essigsäure-isopropenylester.

Auch die Menge an eingesetztem Katalysator beeinflusst wesentlich den Verlauf des erfindungsgemässen Verfahrens. Im allgemeinen beträgt die erforderliche Menge an Katalysator 4 bis 40 Molprozent bezogen auf die Menge eingesetzten Edukts Ia, Ib bzw. Ic.

Die optimale Reaktionstemperatur hängt von verschiedenen Faktoren ab, u.a. von der Natur des verwendeten Lösungsmittels, liegt aber im allgemeinen im Bereich von ca. -20°C bis ca. 50°C, wobei die Dehydratisierung von Ia oder Ib vorzugsweise bei den höheren Temperaturen dieses Bereiches, diejenige von Ic hingegen vorzugsweise bei den niedrigeren Temperaturen erfolgt. Im Falle der Dehydratisierung von Ic in Acetonitril als Lösungsmittel wird das Verfahren vorzugsweise bei ca. 0°C durchgeführt, während die Reaktionstemperatur bei dieser Dehydratisierung in Aceton vorzugsweise Raumtemperatur ist. In beiden Fällen beträgt die Reaktionszeit vorteilhaft von 2 bis 7 Stunden.

Zur Verstärkung der Wirkung des jeweiligen Katalysators wird das erfindungsgemässe Verfahren noch unter Zusatz eines Aminosäure-Hydrobromids, wie beispielsweise Glycin-Hydrobromid, Anthranilsäure-Hydrobromid, Lysin-Hydrobromid oder β-Alanin-Hydrobromid, vorzugsweise das letztgenannte, durchgeführt. Die Menge Aminosäure-Hydrobromid beträgt zweckmässigerweise 1 bis 10 Molprozent, vorzugsweise 1 bis 4 Molprozent, bezogen auf die Menge Edukt. Ein Molverhältnis von ca. 1:1 Calciumbromid-Dihydrat und β-Alanin-Hydrobromid erweist sich als besonders vorteilhaft für die Dehydratisierung.

Im Falle der Verwendung von Calciumbromid oder von dessen Dihydrat als Katalysator erweist sich auch noch das Vorhandensein einer kleinen Menge Essigsäure als besonders vorteilhaft für die Dehydratisierung. Sehr gute Ausbeuten werden beispielsweise durch die Verwendung von ca. 40 Molprozent Calciumbromid-Dihydrat in Acetonitril mitsamt einer Spur Essigsäure bei ca. 0°C erzielt, wobei die optimale Reaktionszeit bei ca. 5 Stunden liegt.

Als Alternative zur Verstärkung der Wirkung von Calciumbromid oder von dessen Dihydrat als Katalysator durch Zusatz eines Aminosäure-Hydrobromids oder von Essigsäure kann man das erfindungsgemässe Verfahren unter Zusatz einer Erde, z.B. Montmorillonit, einer polymeren Säure, z.B. Polymethacrylsäure, oder des Hydrobromidsalzes einer polymergebundenen Aminosäure, z.B. Polyvinylphenylalaninhydrobromid, oder von Calciumglutamat, oder von Glutamin- oder Asparaginsäure durchführen. In allen Fällen mit Ausnahme der Verwendung einer Erde erfolgt die Dehydratisierung zweckmässigerweise in Gegenwart von Acetonitril als Lösungsmittel bei Raumtemperatur. Im Falle der Verwendung einer Erde erfolgt sie zweckmässigerweise in Gegenwart von Acetonitril bei Temperaturen bis ca. 0°C. Die molare Menge des Zusatzes entspricht zweckmässigerweise etwa derjenigen des Katalysators und liegt vorzugsweise im unteren Teil des Bereiches von 4-40 Molprozent bezogen auf die Menge eingesetzten Edukts, insbesondere ca. 4 Molprozent.

Eine besonders bevorzugte Ausführungsform des erfindungsgemässen Verfahrens besteht darin, dass man 1-(9-Acetoxy-3,7-dimethyl-4-hydroxy-nona-2,5,7-trienyl)-2,6,6-trimethyl-cyclohexen in Gegenwart von Calciumbromid-Dihydrat und β-Alanin-Hydrobromid in einem Molverhältnis von ca. 1:1 entweder in Acetonitril bei ca. 0°C oder in Aceton bei Raumtemperatur dehydratisiert.

Die Ausgangsmaterialien Ia, Ib und Ic sind bekannte Verbindungen. Das 1-(9-Acetoxy-3,7-dimethyl-4-hydroxy-nona-2,5,7-trienyl) -2,6,6-trimethylcyclohexen (Ic) kann beispielsweise zusammen mit einer viel kleineren Menge des entsprechenden Diacetats 1-(4,9-Diacetoxy-3,7-dimethyl-nona-2,5,7-trienyl)-2,6,6-trimethyl-cyclohexen durch Transveresterung von Hydroxenin [1-(4,9-Dihydroxy-3,7-dimethyl-nona-2,5,7-trienyl) -2,6,6-trimethyl-cyclohexen] mit Essigester-methylester oder durch Acetylierung mit Essigsäureanhydrid hergestellt werden. Das Produkt dieser Acetylierung besteht üblicherweise aus einem 85:15- bis 92:8-Gemisch des Monoacetats (Ic) und des Diacetats. Das Diacetat lässt sich durch das erfindungsgemässe Verfahren ebenfalls in die Verbindung der Formel IIc überführen, jedoch ist die Reaktionsgeschwindigkeit wesentlich kleiner als diejenige mit dem Monoacetat.

Das Dehydratisierungsprodukt der Formel IIa oder IIb eignet sich als Zwischenprodukt zur Herstellung von Vitamin A, β-Carotin bzw. Zeaxanthin, während das Dehydratisierungsprodukt der Formel IIc Vitamin A-Acetat ist.

Die Erfindung wird durch die folgenden Beispiele veranschaulicht.

### Beispiel 1

### Herstellung von 4-Acetoxy-1-(5-acetoxy-3-methyl-penta-1(E),3(E)-dienyl)-2,6,6-trimethyl-cyclohexen (IIb)

Eine Lösung von 34,2 g 4-Acetoxy-1-(5-acetoxy-3-methyl-penta-1(E),3(E)-dienyl) -2,6,6-trimethyl-cyclohexanol (99%ige Reinheit gemäss Gaschromatographie; 100 mMol) in 160 ml Essigsäure-isopropenylester (1470 mMol) wird unter Argon und Rühren mit 1,46 g Magnesiumbromid-Hexahydrat (5 mMol) versetzt, und das Gemisch wird 15,5 Stunden bei Rückflusstemperatur unter intensivem Rühren (500 U/Min.) erhitzt (Oelbadtemperatur 130-135°C, Innentemperatur 95-97°C). Man verfolgt den Reaktionsverlauf mittels Gaschromatographie. Anschliessend kühlt man das Gemisch auf Raumtemperatur ab und versetzt es mit 5 ml Triäthylamin (36 mMol) und 500 ml Toluol. Dann wird das Gemisch unter vermindertem Druck (Wasserstrahlvakuum) bei 40°C eingeengt, der ölige Rückstand in 400 ml Toluol gelöst und die organische Lösung zweimal mit je 400 ml gesättigter Natriumhydrogencarbonatlösung gewaschen. Man extrahiert die wässrigen Phasen zweimal mit je 400 ml Toluol nach. Die organischen Phasen werden vereinigt, über wasserfreiem Natriumsulfat getrocknet und filtriert, und das Filtrat wird unter vermindertem Druck bei ca. 40°C eingedampft und anschliessend der Rückstand ca. 2 Stunden unter Hochvakuum getrocknet.

Auf diese Weise erhält man ca. 40,0 g 4-Acetoxy-1-(5-acetoxy-3-methylpenta-1(E),3(E)-dienyl) -2,6,6-trimethyl-cyclohexen; Reinheit gemäss Gaschromatographie ca. 71,2 Flächenprozent. Dieses Rohprodukt kann unmittelbar weiterverwendet werden, z.B. als Zwischenprodukt zur Herstellung von Zeaxanthin.

### Beispiel 2

### Herstellung von 1-(9-Acetoxy-3,7-dimethyl-nona-1(E),3(E),5(E),7(E)-tetraenyl)-2,6,6-trimethyl-cyclohexen (IIc) (1. Variante)

34,45 g eines Gemisches von 1-(9-Acetoxy-3,7-dimethyl-4-hydroxy-nona-2(E),5(Z),7(Z) -trienyl)-2,6,6-trimethyl-cyclohexen (Ic) und 1-(4,9-Diacetoxy-3,7-dimethyl-nona-2(E),5(Z),7(Z)-trienyl) -2,6,6-trimethyl-cyclohexen (Id) (99 mMol; Molverhältnis Ic:Id ca. 85:15) werden in 600 ml Acetonitril gelöst. Zur auf 0°C abgekühlten Lösung gibt man 0,48 g Calciumbromid-Dihydrat (2 mMol) sowie 0,34 g β-Alanin-Hydrobromid (2 mMol), und man rührt das Gemisch ca. 3 Stunden. Man verfolgt den Reaktionsverlauf mittels Dünnschichtchromatographie, bis festgestellt wird, dass fast keine Spuren des Ausgangsmaterials noch bleiben.

Anschliessend werden dem Reaktionsgemisch 300 ml halbgesättigte Natriumhydrogencarbonatlösung und 150 ml Methylenchlorid gegeben. Man extrahiert die organische Phase dreimal mit je 100 ml Methylenchlorid und trocknet die vereinigten organischen Phasen mit 50 g wasserfreiem Natriumsulfat. Nach Abfiltration des Trocknungsmittels und Waschen desselben mit 100 ml Methylenchlorid dampfte man das Filtrat unter vermindertem Druck bei einer Badtemperatur von 40°C ein. Der Rückstand wird 30 Minuten bei 45°C/ 66,7 Pa (0,5 mmHg) bis zur Gewichtskonstanz getrocknet. Auf diese Weise erhält man 32,3 g eines Gemisches von 1-(9-Acetoxy-3,7-dimethyl-nona-1(E),3(E),5(E),7(E)-tetraenyl) -2,6,6-trimethyl-cyclohexen (IIc, Vitamin A-Acetat) und dem entsprechenden 13-cis-Isomeren ("13-cis") als gelben Sirup.

| Analyse (HPLC): | |
|---|---|
| IIc | 64,7% |
| 13-cis | 11,5% |

Man löst das Rohprodukt (IIc + 13-cis) in 55 ml eines Lösungsmittelgemisches, das aus 95 ml Methanol, 5 ml Methylenchlorid und 0,1 ml Pyridin vorbereitet wurde. Die Lösung wird mit reinem IIc angeimpft und 16 Stunden bei -25°C gerührt. Anschliessend werden die resultierenden Kristalle abfiltriert und zweimal mit je 12 ml vorgekühltem (-25°C) Methanol gewaschen und 30 Minuten bei 35°C/40 mmHg getrocknet. Auf diese Weise erhält man das 1-(9-Acetoxy-3,7-dimethyl-nona-1(E),3(E),5(E),7(E)-tetraenyl) -2,6,6-trimethylcyclohexen (IIc) in reinerer Form (mit ca. 5-10 Molprozent des 13-cis-Isomeren).

| Analyse (HPLC): | |
|---|---|
| IIc | 91,0% |
| 13-cis | 7,6% |

### Beispiel 3

### Herstellung von 1-(9-Acetoxy-3,7-dimethyl-nona-1(E),3(E),5(E),7(E)-tetraenyl)-2,6,6-trimethyl-cyclohexen (IIc) (2. Variante)

34,45 g eines Gemisches von 1-(9-Acetoxy-3,7-dimethyl-4-hydroxy-nona-2(E),5(Z),7(Z) -trienyl)-2,6,6-trimethyl-cyclohexen (Ic) und 1-(4,9-Diacetoxy-3,7-dimethyl-nona-2(E),5(Z),7(Z)-trienyl) -2,6,6-trimethyl-cyclohexen (Id) (100 mMol; Molverhältnis Ic:Id ca. 85:15) werden in 600 ml Aceton gelöst. Man rührt die Lösung bei Raumtemperatur und gibt 0,96 g Calciumbromid-Dihydrat (4 mMol) sowie 0,68 g β-Alanin-Hydrobromid (4 mMol) zu. Anschliessend wird das Reaktionsgemisch ca. 4 Stunden gerührt.

Danach werden aus dem Gemisch 32,9 g eines Gemisches von 1-(9-Acetoxy-3,7-dimethyl-nona-1(E),3(E),5(E),7(E)-tetraenyl) -2,6,6-trimethylcyclohexen (IIc, Vitamin A-Acetat) und dem entsprechenden 13-cis-Isomeren ("13-cis") als gelber Sirup isoliert, und zwar unter Verwendung der gleichen Aufarbeitungsmethode, wie diese im zweiten Absatz des Beispiels 2 beschrieben ist.

| Analyse (HPLC): | |
|---|---|
| IIc | 51,7% |
| 13-cis | 15,1% |

Man führt auch in diesem Fall diejenige Kristallisationsmethode durch, die im dritten Absatz des Beispiels 2 beschrieben ist, und erhält auf diese Weise das 1-(9-Acetoxy-3,7-dimethyl-nona-1(E),3(E),5(E),7(E)-tetraenyl) -2,6,6-trimethyl-cyclohexen (IIc) in reinerer Form (mit kleinerem Anteil 13-cis).

| Analyse (HPLC): | |
|---|---|
| IIc | 82,6% |
| 13-cis | 7,5% |

## Patentansprüche

1. Verfahren zur Herstellung von Cycloalkenylalkenen mit durchkonjugierter Struktur, dadurch gekennzeichnet, dass man 1-(5-Acetoxy-3-methylpenta-1,3-dienyl) -2,6,6-trimethyl-cyclohexanol oder 4-Acetoxy-1-(5-acetoxy-3-methyl-penta-1,3-dienyl) -2,6,6-trimethyl-cyclohexanol oder 1-(9-Acetoxy-3,7-dimethyl-4-hydroxy-nona-2,5,7-trienyl) -2,6,6-trimethyl-cyclohexen in Gegenwart eines Erdalkalimetallbromids oder von Manganbromid, oder eines Hydrats eines solchen Bromids, als Katalysator sowie in einem organischen Lösungsmittel dehydratisiert.

2. Verfahren nach Anspruch 1, dadurch gekennzeichnet, dass der Katalysator Calciumbromid, Calciumbrornid-Dihydrat, Magnesiumbromid, Magnesiumbromid-Hexahydrat oder Manganbromid ist.

3. Verfahren nach Anspruch 1 oder 2, dadurch gekennzeichnet, dass das Lösungsmittel Acetonitril, Aceton oder Essigsäure-isopropenylester ist.

4. Verfahren nach einem der Ansprüche 1 bis 3, dadurch gekennzeichnet, dass die Menge an eingesetztem Katalysator 4 bis 40 Molprozent bezogen auf die Menge eingesetzten Edukts beträgt.

5. Verfahren nach einem der Ansprüche 1 bis 4, dadurch gekennzeichnet, dass die Dehydratisierung im Temperaturbereich von -20°C bis 50°C durchgeführt wird.

6. Verfahren nach einem der Ansprüche 1 bis 5, dadurch gekennzeichnet, dass die Dehydratisierung unter Zusatz von β-Alanin-Hydrobromid durchgeführt wird.

7. Verfahren nach Anspruch 6, dadurch gekennzeichnet, dass die Menge β-Alanin-Hydrobromid 1 bis 4 Molprozent bezogen auf die Menge Edukt beträgt.

8. Verfahren nach einem der Ansprüche 1 bis 7, dadurch gekennzeichnet, dass die Dehydratisierung in Gegenwart von ca. 40 Molprozent Calciumbromid-Dihydrat in Acetonitril mitsamt einer Spur Essigsäure bei ca. 0°C erfolgt.

9. Verfahren nach Anspruch 1, dadurch gekennzeichnet, dass man 1-(9-Acetoxy-3,7-dimethyl-4-hydroxy-nona-2,5,7-trienyl) -2,6,6-trimethyl-cyclohexen in Gegenwart von Calciumbromid-Dihydrat und β-Alanin-Hydrobromid in einem Molverhältnis von ca. 1:1 in Acetonitril bei ca. 0°C dehydratisiert.

10. Verfahren nach Anspruch 1, dadurch gekennzeichnet, dass man 1-(9-Acetoxy-3,7-dimethyl-4-hydroxy-nona-2,5,7-trienyl) -2,6,6-trimethyl-cyclohexen in Gegenwart von Calciumbromid-Dihydrat und β-Alanin-Hydrobromid in einem Molverhältnis von ca. 1:1 in Aceton bei Raumtemperatur dehydratisiert.

## Claims

1. A process for the manufacture of cycloalkenylalkenes having a through-conjugated structure, characterized by dehydrating 1-(5-acetoxy-3-methyl-penta-1,3-dienyl)-2,6,6-trimethyl-cyclohexanol or 4-acetoxy-1-(5-acetoxy-3-methylpenta-1,3-dienyl)-2,6,6-trimethyl-cyclohexanol or 1-(9-acetoxy-3,7-dimethyl-4-hydroxy-nona-2,5,7-trienyl)-2,6,6-trimethyl-cyclohexene in the presence of an alkaline earth metal bromide or of manganese bromide or of a hydrate of such a bromide as the catalyst and in an organic solvent.

2. A process according to claim 1, characterized in that the catalyst is calcium bromide, calcium bromide dihydrate, magnesium bromide, magnesium bromide hexahydrate or manganese bromide.

3. A process according to claim 1 or 2, characterized in that the solvent is acetonitrile, acetone or isopropenyl acetate.

4. A process according to any one of claims 1 to 3, characterized in that the amount of catalyst used is 4 to 40 mol percent based on the amount of educt used.

5. A process according to any one of claims 1 to 4, characterized in that the dehydration is carried out in the temperature range of -20°C to 50°C.

6. A process according to any one of claims 1 to 5, characterized in that the dehydration is carried out with the addition of β-alanine hydrobromide.

7. A process according to claim 6, characterized in that the amount of β-alanine hydrobromide is 1 to 4 mol percent based on the amount of educt.

8. A process according to any one of claims 1 to 7, characterized in that the dehydration is effected in the presence of about 40 mol percent of calcium bromide dihydrate in acetonitrile together with a trace of acetic acid at about 0°C.

9. A process according to claim 1, characterized in that 1-(9-acetoxy-3,7-dimethyl-4-hydroxy-nona-2,5,7-trienyl)-2,6,6-trimethyl-cyclohexene is dehydrated in the presence of calcium bromide dihydrate and β-alanine hydrobromide in a molar ratio of about 1:1 in acetonitrile at about 0°C.

10. A process according to claim 1, characterized in that 1-(9-acetoxy-3,7-dimethyl-4-hydroxy-nona-2,5,7-trienyl)-2,6,6-trimethyl-cyclohexene is dehydrated in the presence of calcium bromide dihydrate and β-alanine hydrobromide in a molar ratio of about 1:1 in acetone at room temperature.

## Revendications

1. Procédé pour la préparation de cycloalcénylalcènes de structure à conjugaisons accumulées, caractérisé en ce que l'on déshydrate du 1-(5-acétoxy-3-méthylpenta-1,3-diényl)-2,6,6-triméthylcyclohexanol ou du 4-acétoxy-1-(5-acétoxy-3-méthylpenta-1,3-diényl)-2,6,6-triméthylcyclohexanol ou du 1-(9-acétoxy-3,7-diméthyl-4-hydroxynona-2,5,7-triényl)-2,6,6-triméthylcyclohexène en présence d'un bromure de métal alcalino-terreux ou de bromure de manganèse, ou d'un hydrate d'un tel bromure, en tant que catalyseur, ainsi que dans un solvant organique.

2. Procédé selon la revendication 1, caractérisé en ce que le catalyseur est le bromure de calcium, le bromure de calcium dihydraté, le bromure de magnésium, le bromure de magnésium hexahydraté ou le bromure de manganèse.

3. Procédé selon la revendication 1 ou 2, caractérisé en ce que le solvant est l'acétonitrile, l'acétone ou l'acétate d'isopropényle.

4. Procédé selon l'une des revendications 1 à 3, caractérisé en ce que la quantité de catalyseur utilisé va de 4 à 40 % en moles, par rapport à la quantité de produit de départ utilisé.

5. Procédé selon l'une des revendications 1 à 4, caractérisé en ce que la déshydratation est effectuée dans la plage de températures allant de -20 à 50°C.

6. Procédé selon l'une des revendications 1 à 5, caractérisé en ce que la déshydratation est effectuée avec addition de bromhydrate de β-alanine.

7. Procédé selon la revendication 6, caractérisé en ce que la quantité de bromhydrate de β-alanine va de 1 à 4 % en moles par rapport à la quantité de produit de départ.

8. Procédé selon l'une des revendications 1 à 7, caractérisé en ce que la déshydratation est effectuée en présence d'environ 40 % en moles de bromure de calcium dihydraté, dans de l'acétonitrile contenant une trace d'acide acétique, aux environs de 0°C.

9. Procédé selon la revendication 1, caractérisé en ce que l'on déshydrate du 1-(9-acétoxy-3,7-diméthyl-4-hydroxynona-2,5,7-triényl)-2,6,6-triméthylcyclohexène en présence de dihydrate de bromure de calcium et de bromhydrate de β-alanine en un rapport molaire d'environ 1:1, dans de l'acétonitrile, aux environs de 0°C.

10. Procédé selon la revendication 1, caractérisé en ce que l'on déshydrate du 1-(9-acétoxy-3,7-diméthyl-4-hydroxynona-2,5,7-triényl)-2,6,6-triméthylcyclohexène en présence de dihydrate de bromure de calcium et de bromhydrate de β-alanine en un rapport molaire d'environ 1:1, dans de l'acétone, à la température ambiante.
